# EUROPEAN PATENT SPECIFICATION

(11) **EP 1 392 364 B1**
(45) Date of publication and mention of the grant of the patent: **16.11.2011**
(21) Application number: 02754586.2
(22) Date of filing: 24.05.2002
(51) Int. Cl.: A61K 47/32

(54) **LIQUID POLYMER COMPOSITION FOR PREVENTION AND TREATMENT OF THE ORAL CAVITY DISEASES**
FLÜSSIGE POLYMERZUSAMMENSETZUNG ZUR VORBEUGUNG UND BEHANDLUNG VON KRANKHEITEN DER MUNDHÖHLE
COMPOSITION POLYMERE LIQUIDE DESTINEE A LA PREVENTION ET AU TRAITEMENT DES AFFECTIONS DE LA CAVITE BUCCALE

(30) Priority: 25.05.2001 EP 01112751
(43) Date of publication of application: 03.03.2004
(73) Proprietor: Italmed S.N.C. Di Galli G. & Pacini G., 50132 Firenze (IT)
(72) Inventor: GALLI, Giovanna, I-50132 Firenze (IT); PACINI, Gigliola, I-00100 Roma (IT)
(74) Representative: Celestino, Marco
(86) International application number: PCT/EP2002/005728
(87) International publication number: WO 2002/098464

(56) References cited:
- EP-A- 0 404 558
- EP-A- 1 112 750
- DE-A- 4 125 048
- US-A- 4 701 320
- HIGASHI, KIYOTSUGU ET AL: "Local drug delivery systems for the treatment of periodontal disease" J. PHARMACOBIO-DYN. (1991), 14(2), 72-81, XP001017810
- PATIL, G. V. ET AL: "Controlled release of chlorhexidine diacetate from periodontal strips of polymer systems" MACROMOL.--NEW FRONT., PROC. IUPAC INT. SYMP. ADV. POLYM. SCI. TECHNOL. (1998), VOLUME 2, 662-664. EDITOR(S): SRINIVASAN, K. S. V. PUBLISHER: ALLIED PUBLISHERS LTD., NEW DELHI, INDIA., XP001021488
- KITA K ET AL: "PHARMACEUTICAL CHARACTERISTICS OF SUSTAINED-RELEASE GEL OINTMENT FOR PERIODONTAL POCKETS" YAKUZAIGAKU, YAKUJI NIPPO-SHA, TOKYO, JP, vol. 50, no. 3, 1990, pages 239-245, XP001012892 ISSN: 0372-7629

## Description

### Field of the invention

The present invention relates to a pharmaceutical composition for treating diseases of the mouth, in particular periodontal diseases.

### Description of the prior art

A periodontal disease is caused by a pathogenic microbial ecology established within the gingival sulcus which deepens to become a periodontal pocket. This microbial ecology, located deep within the periodontal pocket, differs greatly from that of the superficial oral environment by being more anaerobic, having a larger number of Gram negative organisms, and having a greater proportion of motile species.

Several factors prevent medicinal agents from diffusing when applied to the superficial periodontal tissues. Anatomically, the gum tissue is closely adapted to the neck of the teeth, mechanically restricting the diffusional pathway. In addition, a fluid termed gingival crevice fluid, which has the approximate composition of plasma, permeates the periodontal environment and is continually produced by the diseased periodontal tissues at a rate of 10 to 100 microliters per hour. This fluid, emanating from the diseased pocket lining, creates a net outward flow, which further impedes the introduction of medications by superficially applied drug delivery devices.

These interferences are sufficiently effective to insulate the pocket environment to the extent that saliva does not penetrate, and topically applied medicinal agents have been found largely ineffective in the treatment of established periodontitis. Although mouth rinses may be effective in the reduction of superficial gingivitis resulting from poor home care procedures, the effective radius of action of these agents does not extend to the periodontal pocket.

Introduction of antibacterial agents in solution into the periodontal pocket is also ineffective due to the rapid clearance thereof, so that the duration of contact at the active site is minimal. Conventional therapy for periodontal disease, as first enunciated by Pierre Fauchard in 1746 in his book entitled "The Surgeon Dentist, a Treatise on Teeth", involves the mechanical removal of bacterial plaques and accumulations from the periodontal pocket at periodic intervals. This may include periodontal surgery to achieve access and to recontour damaged tissues. These procedures require a high degree of technical expertise from the practitioners of the art, are expensive, and often result in pain, extensive bleeding, and general local discomfort. Since these procedures provide, at best, only temporary reduction in bacterial populations, they must be repeated at regular intervals to be effective. As discussed by Lindhe and co-workers, in "Healing Following Surgical/Non-Surgical Treatment of Periodontal Disease, a clinical study" in Journal of Clinical Periodontology, vol. 9, pages 115-128, (1982), the frequency of repetition needed for optimal results may be as high as once every two weeks.

Methods for administering drugs for periodontal therapy have heretofore largely been concerned with superficial application. For example:
- long-acting capsules or tablets held in the mouth (US 3,911,099);
- buccal implants for releasing drugs into the saliva (US 4,020,558);
- topically applied gels (US 3,679,360);
- topically applied drug-containing bandages (US 3,339,546);
- a drug containing plastic hardenable mass (US 3,964,164);
- a medicated periodontal dressing (US 3,219,527);
- a topical dressing composed of a finely divided particulate carrier and suspended medicinal agents (US 3,698,392);
- a bandage for covering moist mucosal surfaces (US 3,339,546);
- a microencapsulated liquid droplet formation for topical application to the gums of dogs and other animals (US 4,329,333);
- and foam film devices containing medication (US 3,844,286).

In addition, several fibrous forms for superficial medication have been described, including:
- impregnated or drug-releasing forms of dental floss (US Pat. Nos. 3,417,179, 2,667,443, 2,748,781, 3,942,539);
- solid absorbable fibres of polyglycolic acid with medicates incorporated therein (US 3,991,766);
- and cellulose acetate hollow fibres (US 4,175,326).

Systemic antibiotic therapies for periodontal infections have also been used. In this instance, the objective is to eliminate or suppress any growth of specific pathogenic species. Systemic administration of antibiotics starts by selection of the antibiotic with appropriate antibacterial spectrum. Thus, for example, one might administer penicillin to eliminate Gram positive anaerobe infections, metranidazole to eliminate Gram negative anaerobe infections, and tetracycline to eliminate actinobacillus infections. Actually, specific organisms sensitive to the relatively low concentrations of antibiotic achieved by this therapy (ca. 2-10 ug/ml) are selectively eliminated.

Owing to the low concentrations of antibiotic achieved by systemic administration and the relative high levels of bacterial resistance associated with periodontal pathogens, the clinical success of this therapy has been poor, as discussed by Genco in "Antibiotics in the Treatment of Human Periodontal Diseases", in G. Periodontology, vol. 52, pages 545-558 (1981). Thus, it appears that none of the previously disclosed procedures has led to an acceptable system for delivering optimally effective levels of antibacterial substances to the site of periodontal disease activity.

EP0404558 describes the use of a therapeutic agent delivery device to be placed within the periodontal pocket in such a manner that the diseased pocket regions come in intimate contact with it. The active agent is thus released at the site of disease, eliminating the variability inherent in long diffusional pathways associated with superficial or systemic treatments. The composition has a liquid methacrylic polymer, a therapeutic agent and a release adjusting agent. The polymer retains the therapeutic agent, and the release adjusting agent progressively dissolves the polymer so that the therapeutic agent is slowly released. However, owing to the presence of the release adjusting agent, biocompatibility may be reduced, in particular in presence of bone wounds, as well as allergic problems may arise. Moreover, the addition of an adhesive agent is suggested, since the polymeric film may not adhere to the surface to treat. Polyethylene glycols or dibutyl Phthalate are the preferred plasticizers; such agents play a role in enhancing the rate of degradation of the film and in improving its adherence. A further limit is that the release of the therapeutic agent can last not more than a few hours. In addition, it appears that delivery of optimal concentrations of any medicinal agent to disease sites within the periodontal pocket has not been addressed. The suggested liquid methacrylic polymer are: alcoholic solutions of Eudragit® RL with Eudispert® as release adjusting agent; Eudragit RS+RL in ratio 1/1 with Eudispert as release adjusting agent. Other release adjusting agents are cross-linking agents (such as glutaraldehide, citric acid, lysine, aspartic acid, glutaric acid), polysaccarides (such as dextran), lipids (such as sodium docusate), polyhydroxycompound (such as PEG, glycerol, propylene glycol), protein (such as Byco E or Byco C).

A similar approach is described in DE4125048, wherein Eudragit E is used that forms a polymeric film soluble in water. This way, a controlled release of a therapeutic agent is obtained by dissolution in saliva of the film. However, this mechanism does allow a release of the therapeutic agent in short periods such as a few hours.

Other similar approaches to this problem are described in:
- EP 140766, wherein a polymeric matrix such as ethylene vinyl acetate copolymer and other copolymers or fibres are used which are semi-permeable to a therapeutic agent. The copolymer is put into the periodontal pocket and then removed after a certain time, during which the therapeutic agent has been released. This system has the drawback that the polymeric matrix is semi permeable and not fully permeable. For this reason, it does not adhere to the pocket surface as a film and is used only as biocompatible physical vehicle for the therapeutic agent. Moreover, the polymeric matrix has to be released after a certain time, and this requires a further surgery operation;
- US A3925895 describes a methacrylate polymer used for filling canal roots in which a medicament may be incorporated;

- US A 3956480 describes a treatment of teeth by sorbing onto the tooth surfaces a combination of a cationic germicide and an anionic polymer;
- US A 3846542 relates to an acrylic tooth-filling composition containing boric acid-releasing compound;
- EP A2 0264660 describes the use of a dental material for combating caries and periodontosis.

### Summary of the invention

It is therefore object of the present invention to provide a new composition for treating periodontal disease, by means of a therapeutic agent delivery device placed within the periodontal pocket, such that release of the therapeutic agents occurs in the immediate vicinity of the disease process, without that a release adjusting agent or adhesive agent is present so that biocompatibility or allergic problems cannot arise.

It is another object of the present invention to provide a new composition for treating periodontal disease by means of a therapeutic agent delivery device, wherein a slow release of a therapeutic agent is obtained lasting more than in the prior art, and up to 7-10 days.

It is another object of the present invention to provide a new composition for protecting bacterial growth in the vicinity of buccal wounds, and also inside the cavity, such as after surgery like in a socket after dental extraction, after implants, cysts, gingival abscess, apicectomy, granuloma etc., wherein a mechanical protection on the wounds as well as a barrier to bacteria having is obtained at the same time.

These objects are achieved by a composition according to claim 1.

Preferably, said liquid methacrylate copolymer is a mixture of EUDRAGIT® RS 100 and EUDRAGIT® RL 100.

Preferably the polymer/s is/are provided as a liquid in an alcoholic solution, preferably about 96°. When the polymer is spread on a surface, such as by a little brush, the alcohol evaporates and the polymer forms a polymeric film on that surface.

Alternatively, the polymer/s are provided in a hydroalcoholic solution. In this case, the mixture ethanol and water is 1-20% w/w of water. The presence of this little amount of water is advantageous for enhancing the solubilisation of the therapeutic agent when the latter has not high solubility in alcohol, and on the other hand it has a good solubility in water. In this way the preparation of the composition by mixing the polymeric solution and the therapeutic agent (i.e. powder) is faster. Up to 20% of water both the polymeric solution and the final liquid composition remain as a clear solution, i.e. without precipitation of the polymer/s.

Moreover, the liquid polymer can be injected directly into the periodontal pocket, forming a film in situ and releasing the active agent according to the invention in a controlled manner over a desired period of time.

Chemically, EUDRAGIT® RS and RL are copolymers of acrylic and methacrylic acid esters with a low content in quaternary ammonium groups. The ammonium groups are present as salts and make the polymers permeable. The polymers are described in USP/NF as "ammonium methacrylate copolymer, type A" (EUDRAGIT® RL) and "type B" (EUDRAGIT® RS).

A first advantage of the composition according to the invention is that if a therapeutic agent that is soluble both in water and in alcohol is used, within the biocompatible polymeric comprising EUDRAGIT® RL or EUDRAGIT® RS or a mixture thereof, the polymeric film surprisingly adheres to the surfaces and no adhesive additives are necessary. This increases substantially biocompatibility with respect to the prior art, since no adhesive material is necessary, that is instead required in EP0404558. Moreover, this adhesivity does not require that a large amount of polymer is used up to filling the periodontal pockets or other cavities, thus avoiding the need of removing the device later. This allows the cavity to collapse spontaneously and the film to be dissolved naturally in the mouth environment.

A main advantage of the present invention that enhances biocompatibility is that no release adjusting agent is necessary, that is instead required in EP0404558. This result is given by the synergic combination of biocompatible polymeric material, according to claim 1.

More precisely, the film of biocompatible polymeric material is not soluble into water and then it is durable up to a normal consumption within the mouth environment, several days to several weeks, depending on the quantity. If in the film a therapeutic agent is present that is soluble in alcohol but not in water, when water present in the mouth tissues permeates through the film, the therapeutic agent is not released. On the other hand, if a therapeutic agent is not soluble in alcohol, it cannot be accepted within the polymer alcoholic solution.

Therefore, according to claim 1, if in the film of biocompatible polymeric material a therapeutic agent is present that is soluble both in alcohol and in water, when water present in the mouth tissues permeates through the polymeric material, the therapeutic agent is released. The release rate is dependent from the permeability to water of the polymeric material. Mixtures may be obtained from which the therapeutic agent will diffuse at a controlled rate over selected periods of time.

More precisely, by proper selection of the two polymeric material EUDRAGIT® RS and RL, a precise release rate is obtained. In fact, by varying the ratio RS/RL the hydrophilic property varies accordingly. In particular, if RL is increased, the release rate of the therapeutic agent is increased. According to claim 1 the ratio RS/RL is comprised between 1,5/1 and 3/1, so that the release of the therapeutic agent lasts up to 7-10 days. The preferred ratio RS/RL is 2/1.

According to claim 1, the therapeutic agent that is soluble both in water and in alcohol is chosen among:
*Antibacterial Agents* - chlorhexidine acetate, thimerosal, cetylpirydinium chloride, benzalkonium chloride, cetrimide, benzethonium chloride;
*Antibiotics -* piperacillin sodium, carbenicillin sodium, carindacillin sodium, chloramphenicol sodium succinate, clindamycin palmitate hydrochloride, cloxacillin sodium, erythromycin gluceptate and lactobionate, flucloxacillin sodium, lincomycin hydrochloride, nafcillin sodium, tetracycline hydrochloride, minocylcline;
*Dentinal desensitizing agents -* strontium chloride, zinc chloride, calcium chloride, magnesium chloride, tin chloride, potassium sorbate;
*Antivirals -* acyclovir, idoxouridine, amantadine;
and mixtures thereof.

A useful adjuvant procedure to the surgical treatment to prevent or to treat infections of the above mentioned diseases is to put into the cavity a sponge of collagen, alginate, hyaluronate with liquid polymer.

It is recognized that in certain forms of therapy, combinations of these agents in the same delivery system may be useful in order to obtain an optimal effect. Thus, for example, an antibacterial and an antiinflammatory agent may be combined in a single delivery system to provide combined effectiveness.

A further advantage deriving from the use of this new composition according to the invention is the following. Since the volume of distribution is limited to the total volume of gingival crevice fluid produced within the periodontal pocket, relatively high concentrations of therapeutic agent are developed in the pocket. However, the overall amount of therapeutic agent required under these conditions is low, typically a few milligrams. This small amount greatly reduces the effect of the therapeutic agent same at distal sites within the body, thereby greatly decreasing the potential for systemic side effects. By establishing local concentrations of an antibacterial agent sufficient to inhibit growth of all bacteria within the pocket, development of drug resistant strains is minimized. The potential for encouraging the development of drug-resistant pathogens is further minimized by the relatively short duration to achieve the desired effect, typically about three to ten days. By this procedure, the therapeutic agent delivery according to the invention, corresponding to a few milligrams of therapeutic agent, gives effects greater than those expected by the same drug used at much higher dosage by other routes of distribution. This principle results in an unexpectedly high degree of effectiveness from the comparatively small amount of drug utilized. The use of a periodontal or other mechanical retaining and/or diffusion-limiting device further enhances therapeutic effectiveness.

Not limitative examples of the present invention are the following:

### EXAMPLE 1

### Solution

Eudragit® RL 100 4,5% (w/w)
Eudragit® RS 100 7,5% (w/w)
Ethanol 96% q.s. 100 g

### Powder

Piperacillin sodium 10% (w/w)

### EXAMPLE 2

### Solution

Eudragit® RL 100 4,5% (w/w)
Eudragit® RS 100 7,5% (w/w)
Purified water 18% (w/w)
Ethanol 96% q.s. 100 g

### Powder

Piperacillin sodium 10% (w/w)

### EXAMPLE 3

### Solution

Eudragit® RL 100 4,5% (w/w)
Eudragit® RS 100 7,5% (w/w)
Ethanol 96% q.s. 100 g

### Powder

Chloramphenicol sodium succinate 10% (w/w)

### EXAMPLE 4

### Solution

Eudragit® RL 100 4,5% (w/w)
Eudragit® RS 100 7,5% (w/w)
Ethanol 96% q.s. 100 g

### Powder

Clindamycin palmitate 10% (w/w)

### EXAMPLE 5

Eudragit® RL100 4,5% (w/w)
Eudragit® RS100 7,5% (w/w)
Cetrimide 0,5% (w/w)
Chlorexidine acetate 0,2% (w/w)
Ethanol 96% q.s. 100 g

### EXAMPLE 6

Acyclovir 3% (w/w)
Eudragit® RL100 4,5% (w/w)
Eudragit® RS100 7,5% (w/w)
Transcutol 10% (w/w)
Ethanol 96% q.s. 100 g

This composition is used for treating diseases caused by Herpes Labialis.

The foregoing description in the form of examples will so fully reveal the invention according to the conceptual point of view, so that others, by applying current knowledge, will be able to modify and/or adapt for various applications such examples without further research and without parting from the invention, and it is therefore to be understood that such adaptations and modifications will have to be considered as equivalents to the specific examples. The means and the materials to realise the different functions described herein could have a different nature without, for this reason, departing from the field of the invention. It is to be understood that the phraseology or terminology employed herein is for the purpose of description and not of limitation.

## Claims

1. A composition for prevention and treatment of oral cavity diseases, consisting of a therapeutic agent in a biocompatible polymeric material,
wherein
said therapeutic agent is soluble both in water and in alcohol,
said biocompatible polymeric material is a liquid alcoholic solution of a methacrylate copolymer formed by a mixture of EUDRAGIT® RL and EUDRAGIT® RS,
the ratio EUDRAGI® RS/ EUDRAGIT® RL is comprised between 1,5/1 and 3/1,
said biocompatible polymeric material and said therapeutic agent are adapted to be spread in topical combination in such a way that a film is formed topically and said therapeutic agent is then released progressively by water permeation through said polymeric material of said film, and
wherein said therapeutic agent is chosen among:
antibacterial agents selected from - chlorhexidine acetate, thimerosal, cetylpyridinium chloride, benzalkonium chloride, cetrimide, benzethonium chloride;
antibiotics selected from - piperacillin sodium, carbenicillin sodium carindacillin sodium, chloramphenicol sodium succinate, clindamycin palmitate hydrochloride, cloxacillin sodium, erythromycin gluceptate and lactobionate, flucloxacillin sodium, lincomycin hydrochloride, nafcillinsodium, tetracycline hydrochloride, minocycline;
dentinal desensitising agents selected from - strontium chloride, zinc chloride, calcium chloride, magnesium chloride tin chloride, potassium sorbate;
antivirals selected from - acyclovir, idoxuridine, amantadine,
and mixtures thereof.

2. Composition according to claim 1, wherein said liquid methacrylate copolymer is a mixture of EUDRAGIT®RS 100 and EUDRAGIT®RL 100.

3. Composition according to claim 1, wherein said alcoholic solution of said liquid methacrylate copolymer comprises an alcoholic solvent and also 1-20% of water.

4. Composition according to claim 1, wherein said biocompatible polymeric material and said therapeutic agent are mixed in the following weight ratio
Eudragit RL® 100 0,3-5% (w/w)
Eudragit RS® 100 0,5-10% (w/w)
Therapeutic agent 1-20% (w/w)
Ethanol 96% q.s. 100 g

5. Composition according to claim 1, wherein said biocompatible polymeric material and said therapeutic agent are mixed in the following weight ratio
Eudragit RL® 100 0,3-5% (w/w)
Eudragit RS® 100 0,5-10% (w/w)
Therapeutic agent 1-20% (w/w)
Purified water 1-20% (w/w)
Ethanol 96% q.s. 100 g

6. Composition according to claim 1, wherein said Therapeutic agent is chosen among: Piperacillin sodium; Cholramphenicol sodium succinate; Clindamycin palmitate.

7. Composition according to claim 1, wherein said biocompatible polymeric material and said therapeutic agent are mixed in the following weight ratio
Eudragit RL® 100 0,3-5% (w/w)
Eudragit RS® 100 0,5-10% (w/w)
Cetrimide 0,1-1%(w/w)
Chlorexidine acetate 0,05-0,5% (w/w)
Ethanol 96% q.s. 100 g

8. Composition according to claim 1, for treating diseases caused by Herpes Labialis, wherein said biocompatible polymeric material and therapeutic agents are mixed in the following weight ratio
Acyclovir 1-5% (w/w)
Eudragit RL100® 0,3-5% (w/w)
Eudragit RS100® 0,5-10% (w/w)
Transcutol 1-15% (w/w)
Ethanol 96% q.s. 100 g

## Patentansprüche

1. Zusammensetzung zur Prävention und Behandlung von Krankheiten der Mundhöhle, die aus einem therapeutischen Mittel in einem biologisch kompatiblen Polymermaterial besteht,
wobei
das therapeutische Mittel sowohl in Wasser als auch in Alkohol löslich ist,
das biologisch kompatible Polymermaterial eine flüssige alkoholische Lösung eines Methacrylatcopolymers, das durch ein Gemisch von EUDRAGIT® RL und EUDRAGIT® RS gebildet ist, ist,
das Verhältnis EUDRAGIT® RS/EUDRAGIT® RL zwischen 1,5/1 und 3/1 liegt,
das biologisch kompatible Polymermaterial und das therapeutische Mittel so angepasst sind, dass sie in einer topischen Kombination derart ausgebreitet werden, dass topisch ein Film gebildet wird und das therapeutische Mittel dann durch Permeation von Wasser durch das Polymermaterial des Films fortschreitend freigesetzt wird,
und
das therapeutische Mittel aus:
antibakteriellen Mitteln, die aus Chlorhexidinacetat, Thimerosal, Cetylpyridiniumchlorid, Benzalkoniumchlorid, Cetrimid, Benzethoniumchlorid ausgewählt sind;
Antibiotika, die aus Piperacillin-Natrium, Carbenicillin-Natrium, Carindacillin-Natrium, Chloramphenicol-Natriumsuccinat, Clindamycinpalmitathydrochlorid, Cloxacillin-Natrium, Erythromycingluceptat und -lactobionat, Flucloxacillin-Natrium, Lincomycinhydrochlorid, Nafcillin-Natrium, Tetracyclinhydrochlorid, Minocyclin ausgewählt sind; Dentin-Desensibilisierungsmittel, die aus Strontiumchlorid, Zinkchlorid, Calciumchlorid, Magnesiumchlorid, Zinnchlorid, Kaliumsorbat ausgewählt sind;
antiviralen Mitteln, die aus Acyclovir, Idoxuridin,
Amantadin ausgewählt sind,
und Gemischen hiervon, ausgewählt ist.

2. Zusammensetzung nach Anspruch 1, wobei das flüssige Methacrylatcopolymer ein Gemisch von EUDRAGIT® RS 100 und EUDRAGIT® RL 100 ist.

3. Zusammensetzung nach Anspruch 1, wobei die alkoholische Lösung des flüssigen Methacrylatcopolymers ein Alkohollösemittel und ferner 1-20 % Wasser umfasst.

4. Zusammensetzung nach Anspruch 1, wobei das biologisch kompatible Polymermaterial und das therapeutische Mittel in dem im Folgenden angegebenen Gewichtsverhältnis gemischt sind:
Eudragit RL® 100 0,3-5 % (Gew./Gew.)
Eudragit RS® 100 0,5-10 % (Gew./Gew.)
Therapeutisches Mittel 1-20 % (Gew./Gew.)
96%iges Ethanol q.s. 100 g.

5. Zusammensetzung nach Anspruch 1, wobei das biologisch kompatible Polymermaterial und das therapeutische Mittel in dem im Folgenden angegebenen Gewichtsverhältnis gemischt sind:
Eudragit RL® 100 0,3-5 % (Gew./Gew.)
Eudragit RS® 100 0,5-10 % (Gew./Gew.)
Therapeutisches Mittel 1-20 % (Gew./Gew.)
Gereinigtes Wasser 1-20 % (Gew./Gew.)
96%iges Ethanol q.s. 100 g.

6. Zusammensetzung nach Anspruch 1, wobei das therapeutische Mittel aus Piperacillin-Natrium, Chloramphenicol-Natriumsuccinat, Clindamycinpalmitat ausgewählt ist.

7. Zusammensetzung nach Anspruch 1, wobei das biologisch kompatible Polymermaterial und das therapeutische Mittel in dem im Folgenden angegebenen Gewichtsverhältnis gemischt sind:
Eudragit RL® 100 0,3-5 % (Gew./Gew.)
Eudragit RS® 100 0,5-10 % (Gew./Gew.)
Cetrimid 0,1-1 % (Gew./Gew.)
Chlorhexidinacetat 0,05-0,5 % (Gew./Gew.)
96%iges Ethanol q.s. 100 g.

8. Zusammensetzung nach Anspruch 1 zur Behandlung von durch Herpes labialis verursachten Krankheiten, wobei das biologisch kompatible Polymermaterial und das therapeutische Mittel in dem im Folgenden angegebenen Gewichtsverhältnis gemischt sind:
Acyclovir 1-5 % (Gew./Gew.)
Eudragit RL® 100 0,3-5 % (Gew./Gew.)
Eudragit RS® 100 0,5-10 % (Gew./Gew.)
Transcutol 1-15 % (Gew./Gew.)
96%iges Ethanol q.s. 100 g.

## Revendications

1. Composition destinée à la prévention et au traitement des maladies de la cavité buccale, consistant en un agent thérapeutique dans un matériau polymère biocompatible,
dans laquelle
ledit agent thérapeutique est soluble à la fois dans l'eau et dans l'alcool,
ledit matériau polymère biocompatible est une solution alcoolique liquide d'un copolymère de méthacrylate formé par un mélange d'EUDRAGIT® RL et d'EUDRAGIT® RS,
le rapport EUDRAGTT® RS/EUDRAGIT® RL est compris entre 1,5/1 et 3/1,
ledit matériau polymère biocompatible et ledit agent thérapeutique sont adaptés pour être étalés en combinaison topique de telle sorte qu'un film se forme topiquement et ledit agent thérapeutique est alors libéré progressivement par la perméation de l'eau à travers ledit matériau polymère dudit film, et
dans laquelle ledit agent thérapeutique est choisi parmi :
les agents antibactériens choisis parmi l'acétate de chlorhexidine, le thimérosal, le chlorure de cétylpyridinium, le chlorure de benzalkonium, le cétrimide, le chlorure de benzéthonium ;
les antibiotiques choisis parmi la pipéracilline sodique, la carbénicilline sodique, la carindacilline sodique, le succinate sodique de chloramphénicol, le chlorhydrate de palmitate de clindamycine, la cloxacilline sodique, le gluceptate et le lactobionate d'érythromycine, la flucloxacilline sodique, le chlorhydrate de lincomycine, la nafcilline sodique, le chlorhydrate de tétracycline, la minocycline ;
les agents de désensibilisation de la dentine choisis parmi le chlorure de strontium, le chlorure de zinc, le chlorure de calcium, le chlorure de magnésium le chlorure d'étain, le sorbate de potassium ;
les antiviraux choisis parmi l'acyclovir, l'idoxuridine, l_{'}amantadine, et leurs mélanges.

2. Composition selon la revendication 1, dans laquelle ledit copolymère de méthacrylate liquide est un mélange d'EUDRAGIT® RS 100 et d'EUDRAGIT® RL 100.

3. Composition selon la revendication 1, dans laquelle ladite solution alcoolique dudit copolymère de méthacrylate liquide comprend un solvant alcool ainsi que 1 à 20 % d'eau.

4. Composition selon la revendication 1, dans laquelle ledit matériau polymère biocompatible et ledit agent thérapeutique sont mélangés dans le rapport en poids suivant
Eudragit® RL 100 0,3 à 5 % (p/p)
Eudragit® RS 100 0,5 à 10 % (p/p) agent thérapeutique 1 à 20 % (p/p) éthanol 96 % q.s.p. 100 g.

5. Composition selon la revendication 1, dans laquelle ledit matériau polymère biocompatible et ledit agent thérapeutique sont mélangés dans le rapport en poids suivant
Eudragit® RL 100 0,3 à 5 % (p/p)
Eudragit® RS 100 0,5 à 10 % (p/p)
agent thérapeutique 1 à 20 % (p/p)
eau purifiée 1 à 20 % (p/p)
éthanol 96 % q.s.p. 100 g.

6. Composition selon la revendication 1, dans laquelle ledit agent thérapeutique est choisi parmi :
la pipéracilline sodique ; le succinate sodique de chloramphénicol ; le palmitate de clindamycine.

7. Composition selon la revendication 1, dans laquelle ledit matériau polymère biocompatible et ledit agent thérapeutique sont mélangés dans le rapport en poids suivant
Eudragit® RL 100 0,3 à 5 % (p/p)
Eudragit® RS 100 0,5 à 10 % (p/p)
cétrimide 0,1 à 1 % (p/p)
acétate de chlorhexidine 0,05 à 0,5 % (p/p)
éthanol 96 % q.s.p. 100 g.

8. Composition selon la revendication 1, destinée au traitement de maladies provoquées par l'herpès labial, dans laquelle ledit matériau polymère biocompatible et les agents thérapeutiques sont mélangés dans le rapport en poids suivant
acyclovir 1 à 5 % (p/p)
Eudragit® RL 100 0,3 à 5 % (p/p)
Eudragit® RS 100 0,5 à 10 % (p/p)
transcutol 1 à 15 % (p/p)
méthanol 96 % q.s.p. 100 g.
